# EUROPEAN PATENT SPECIFICATION

(11) **EP 1 418 240 B1**
(45) Date of publication and mention of the grant of the patent: **27.05.2009**
(21) Application number: 02028018.6
(22) Date of filing: 13.12.2002
(51) Int. Cl.: C12Q 1/68, C12Q 1/26, C12Q 1/28, C12Q 1/42, C12Q 1/48

(54) **A method and kit for analyzing a target nucleic acid fragment**
Verfahren und Kit zur Analyse eines Ziel-Nucleinsäurefragmentes
Procédé et trousse pour analyser un fragment d'acide nucléique cible

(30) Priority: 06.11.2002 JP 2002322082
(43) Date of publication of application: 12.05.2004
(73) Proprietor: FUJIFILM Corporation, Minato-ku Tokyo (JP)
(72) Inventor: Makino, Yoshihiko, Asaka-shi, Saitama 351-8585 (JP); Mori, Toshihiro, Asaka-shi, Saitama 351-8585 (JP); Iwaki, Yoshihide, Asaka-shi, Saitama 351-8585 (JP)
(74) Representative: Albrecht, Thomas

(56) References cited:
- EP-A- 0 663 447
- US-A- 5 849 487
- PATENT ABSTRACTS OF JAPAN vol. 1996, no. 01, 31 January 1996 (1996-01-31) & JP 07 231799 A (EIKEN CHEM CO LTD;OTHERS: 01), 5 September 1995 (1995-09-05)

### SEQUENCE LISTING

<110> FUJI PHOTO FILM CO., LTD.
<120> A METHOD FOR ANALYZING A TARGET NUCLEIC ACID FRAGMENT AND A KIT FOR ANALYZING A TARGET NUCLEIC ACID FRAGMENT
<130> 12986EP
<140>
   <141>
<150> 322082/2002
   <151> 2002-11-06
<160> 10
<170> PatentIn Ver. 2.1
<210> 1
   <211> 27
   <212> DNA
   <213> Artificial Sequence
<220>
   <223> Description of Artificial Sequence:primer
<400> 1
   gatcagcaag cagctgggat acacgtg 27
<210> 2
   <211> 21
   <212> DNA
   <213> Artificial Sequence
<220>
   <223> Description of Artificial Sequence:primer
<400> 2
   ctgtagcttc ccgttgcggt g 21
<210> 3
   <211> 25
   <212> DNA
   <213> Artificial Sequence
<220>
   <223> Description of Artificial Sequence:primer
<400> 3
   caggcataca ctgaagtgaa aactg 25
<210> 4
   <211> 20
   <212> DNA
   <213> Artificial Sequence
<220>
   <223> Description of Artificial Sequence:primer
<400> 4
   aggtcctgaa cttccagcag 20
<210> 5
   <211> 27
   <212> DNA
   <213> Artificial Sequence
<220>
   <223> Description of Artificial Sequence:primer
<400> 5
   gcgatgctgt aatgactctc gacaagc 27
<210> 6
   <211> 25
   <212> DNA
   <213> Artificial Sequence
<220>
   <223> Description of Artificial Sequence:primer
<400> 6
   ggtctgcaaa ttctgcggcg tcgtc 25
<210> 7
   <211> 19
   <212> DNA
   <213> Artificial Sequence
<220>
   <223> Description of Artificial Sequence:primer
<400> 7
   aacgaagccc agcaaatga 19
<210> 8
   <211> 20
   <212> DNA
   <213> Artificial Sequence
<220>
   <223> Description of Artificial Sequence:primer
<400> 8
   gggctgcagg catacacaga 20
<210> 9
   <211> 19
   <212> DNA
   <213> Artificial Sequence
<220>
   <223> Description of Artificial Sequence:primer
<400> 9
   aacgaagccc agcaaatga 19
<210> 10
   <211> 20
   <212> DNA
   <213> Artificial Sequence
<220>
   <223> Description of Artificial Sequence:primer
<400> 10
   gggctgcagg catacacaaa 20

## Description

### Technical Field

The present invention relates to a method for analyzing a target nucleic acid fragment having a certain nucleotide sequence that is useful in, for example, the clinical examination of infectious diseases caused by viruses, bacteria and the like and the examination of genetic diseases resulting from genetic feature of individual, and a kit for analyzing a nucleic acid fragment using the method. More particularly, the present invention relates to a method for simply analyzing the existence or abundance of a target nucleic acid fragment or the nucleotide sequence of the target nucleic acid fragment by detecting whether or not a polymerase elongation reaction using a target nucleic acid as a template has been proceeded by means of colorimetry, preferably by means of a dry analytical element; and a kit for analyzing a nucleic acid fragment using the method. The present invention relates to a dry analytical element for quantifying pyrophosphoric acid in a sample.

### Background Art

Heretofore, in the clinical examination of infectious diseases caused by viruses, bacteria and the like, specimens have been cultured using body fluids such as blood, feces, expectoration and the like as a sample to identify pathogens such as viruses and bacteria. These methods, however, require a very long period of time to culture specimens, or culturing itself cannot be successfully carried out in the case of some types of viruses or bacteria. Since these methods require special techniques to culture specimens, these methods cannot always provide satisfactory results in a quick and simple way.

A method for identifying pathogens such as viruses and bacteria using the antigen-antibody reaction has also been carried out. This method is a good method in terms of swiftness and simplicity since the examination can be automated. In the antigen-detecting method for detecting a pathogen as an antigen, however, an insufficient amount of a pathogen existing in the sample may result in failure in detection of the pathogen, and this method has a problem in terms of its sensitivity. There is also the problem that the determination of the antigen site which is peculiar to the type of a pathogen is difficult. In contrast, in a method for detecting an antibody which has been produced in a body as a result of pathogen infection, it would take some time from pathogen infection to antibody production, and thus there is a problem that detection cannot be carried out during that period.

On the contrary, a method for detecting a nucleic acid fragment (a target nucleic acid fragment) having a nucleotide sequence peculiar to the type of pathogens such as viruses or bacteria by utilizing complementarity of the nucleotide sequence enables the direct identification of the pathogen. This method is widely employed as a method for examining genes such as the DNA probe method or polymerase chain reaction (PCR). For example, a method for examining hepatitis C virus (HCV) genes is very useful as a method for directly measuring the amount of HCV in considering the interferon (INF) administration in INF therapy for hepatitis C and monitoring of recovery.

Further, genotypes of pathogens such as viruses and bacteria will be elucidated, and development of a novel therapeutic agent using the genotype can be expected. In that case, not only identification of the pathogen but also discovery of the genotype of the pathogen is very important. Genetic screening is indeed an examination method which can satisfy such a demand.

In genetic screening, the individual's genotype can be directly detected without limitation to identification of the pathogen, and thus, it is applicable to the detection of genetic mutation as a cause of genetic diseases and the detection of genetic factors which affect the susceptibility to diseases, for example, life-style related diseases such as cancer and diabetes. In particular, after the entire nucleotide sequence of the human genome was determined, the correlation between the genotype and the disease is further elucidated as a post-genome research, and further development of therapeutic agents utilizing the genotype can be expected. As the post-genome research proceeds, it seems likely that a demand for genetic examination method will increase more and more in the future.

However, currently performed genetic examination methods require special techniques, complicated operations, special apparatuses and the like. Accordingly, facilities capable of conducting genetic examination method are limited to large-scale examination centers and the like. In the examination of infectious diseases caused by viruses and bacteria and also in the examination of the genotype of individual, if diagnosis and determination of the treatment guideline can be carried out on site as early as possible, genetic examination is more effective. Thus, novel genetic examination methods are required with which anyone can perform the detection in simple operations and can swiftly obtain the examination results.

A genetic examination method has been heretofore developed which utilizes the detection of the progress in the polymerase elongation reaction using a target nucleic acid fragment as a template for the purpose of improving simplicity and swiftness. When amplifying a specific nucleic acid region of the target nucleic acid fragment by PCR, a method for detecting a generation process of amplification products as changes in fluorescence intensity in real-time (Real-Time PCR) is a good method in terms of swiftness since it does not require a process of subjecting the amplification product to electrophoresis after PCR and analyzing the result, and it is commercialized as the TaqMan probe method (PE Biosystems) and the Molecular Beacon method (Stratagene). However, these methods utilize fluorescence resonance energy transfer (FRET), and have a problem that they require an apparatus capable of measuring changes in fluorescence intensity and a special hybridization probe labeled with a fluorescent dye and its quencher in combination. Thus, these methods are still in the range of special techniques.

A method for detecting changes in the fluorescence intensity at the time of amplification by PCR of a specific nucleic acid region of the target nucleic acid fragment in the presence of an intercalator fluorescent substance (intercalation monitoring PCR (IM-PCR)) is described in "Igaku no Ayumi (Journal of Clinical and Experimental Medicine)" Vol. 173, No. 12, 1995. This method is good as Real-Time PCR because it does not require any special hybridization probe, but this method still requires an apparatus capable of measuring changes in the fluorescence intensity for its implementation. Regardless the occurrence of PCR amplification of the specific nucleic acid region of the target nucleic acid fragment, the intercalator fluorescent substance binds to all the nucleic acid fragments existing in the system, and thus, it is disadvantageous in terms of specificity.

On the other hand, a method of hybridizing an oligonucleotide primer having nuclease resistance with a specific region of the target nucleic acid fragment, repeating elongation reaction and decomposition reaction in the presence of deoxynucleoside triphosphate (dNTP), DNA polymerase and nuclease, and detecting pyrophosphoric acid or deoxynucleoside monophosphate which was generated, is disclosed in Japanese Patent Publication Laid-Open No. 7-231799. A method for detecting a target nucleic acid fragment by detecting pyrophosphoric acid generated upon polymerase elongation reaction is excellent in that the target nucleic acid fragment can be detected by detecting a general chemical substance which is a by-product of polymerase elongation reaction.

However, a method for a simple detection of pyrophosphoric acid has not yet known, and the above-mentioned publication merely describes a method of detecting luminescence generated upon the reaction between adenosinetriphosphate (ATP) and luciferin in the presence of luciferase, in which the adenosinetriphosphate (ATP) is generated by reacting pyrophosphoric acid with adenosine-5'-phosphosulfate and adenosinetriphosphate (ATP) sulfurylase. This method is disadvantageous in terms of simplicity due to the necessity of an apparatus capable of measuring luminescence. This method is different from the method of the present invention in that it is carried out under conditions under which the elongation reaction does not proceed in a substantially continuous manner, since this method uses a primer having nuclease resistance, uses DNA polymerase in combination with nuclease, and repeats polymerase reaction and nuclease reaction

### Disclosure of the Invention

An object of the present invention is to provide a method for analyzing a target nucleic acid fragment which can be simply and swiftly carried out by anyone using a small apparatus without requiring any special techniques, complicated operations, and special apparatuses. In order to achieve this object, it is another object of the present invention to provide a method for analyzing a target nucleic acid fragment which can be space-saving and automated. Further, it is another object to provide a kit for analyzing a target nucleic acid fragment using these methods for analysis. It is further another object of the present invention is to provide a dry analytical element for quantifying pyrophosphoric acid in a sample which enables quantification of pyrophosphoric acid in a small amount of sample by colorimetry and is excellent in convenience and quickness.

The invention relates to a dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus, which comprises a reagent layer containing xanthosine or inosine, pyrophosphatase, purine nucleoside phosphorylase, xanthine oxidase, peroxidase, and a color developer.

Further, the invention relates to a method for analyzing a target nucleic acid fragment which comprises steps of reacting a target nucleic acid fragment, at least a part of its nucleotide sequence being known, at least one primer complementary with a part of the target nucleic acid fragment, at least one deoxynucleoside triphosphate (dNTP) and at least one polymerase, and detecting occurrence of the progress on polymerase elongation reaction using the target nucleic acid fragment as template and starting from the 3' terminus of the primer, wherein whether or not polymerase elongation reaction is proceeded is detected by detecting pyrophosphoric acid generated upon said polymerase elongation reaction, wherein detection of pyrophosphoric acid is carried out using colorimetry and a dry analytical element as defined above.

According to another aspect, the invention relates to a kit which comprises at least one primer complementary with a part of the target nucleic acid fragment to be analyzed, at least one deoxynucleoside triphosphate, at least one polymerase, and a dry analytical element as defined above for quantifying pyrophosphoric acid.

Finally, the invention relates to a kit which comprises at least one primer complementary with a part of the target nucleic acid fragment to be analyzed, at least one deoxynucleoside triphosphate (dNTP), at least one polymerase, pyrophosphatase, and a dry analytical element as defined above for quantifying inorganic phosphorus.

### Brief Description of the Drawings

Fig. 1 is a conceptual view illustrating an embodiment of the present invention.
Fig. 2 is a perspective view exemplifying a kit in the form of a cartridge according to the present invention.
Fig. 3 is a perspective view showing a construction of a system when a kit in the form of a cartridge according to the present invention is used.
Fig. 4 shows a correlation between the number of *Pseudomonas aeruginosa* in human whole blood and changes in the optical density of reflection (OD_{R}) with time.
Fig. 5 shows a correlation between the number of *Pseudomonas aeruginosa* in human whole blood and the optical density of reflection (OD_{R}) 5 minutes later.
Fig. 6 shows a correlation between the number of *Pseudomonas aeruginosa* in human whole blood and the optical density of reflection (OD_{R}) 5 minutes later.
Fig. 7 shows a correlation between the active form/inactive form of ALDH-2 of a sample and changes in the optical density of reflection (OD_{R}) with time.
Fig. 8 shows a magnitude correlation between the AHDH-2 type of the sample / primer type and the optical density of reflection (OD_{R}) 5 minutes later.

Regarding numerical references in these drawings, 10 represents a kit, 21 a substrate, 22 a lid, 31 an opening portion, 32 a reaction cell, 33 a detecting unit, 41 a canaliculus, 51 a dry analytical element, 61 a temperature control unit, 62 a temperature control unit, 71 a detection unit, 72 a detection unit, 81 a primer, 82 deoxynucleoside triphosphate (dNTP), and 83 polymerase.

### Detailed Description of the Invention

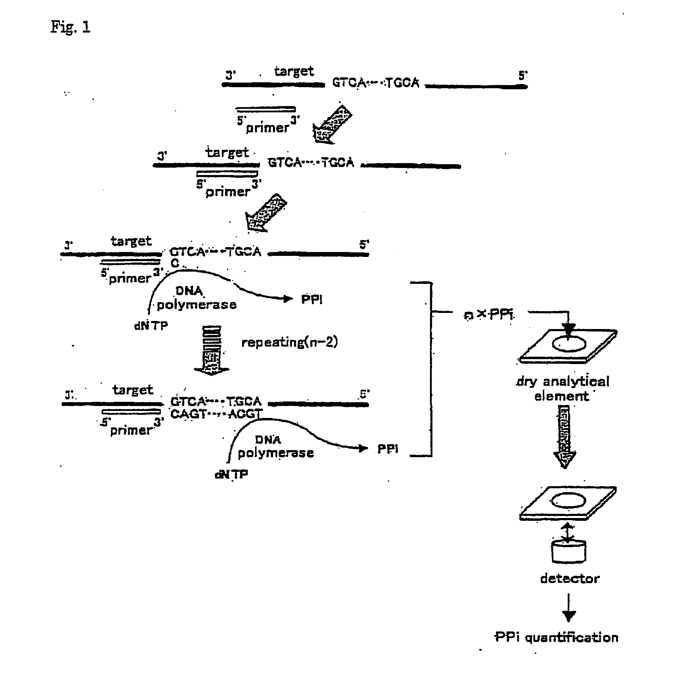

According to a preferred embodiment of the present invention, the method for analyzing a target nucleic acid fragment is carried out by analyzing pyrophosphoric acid by colorimetry, and pyrophosphoric acid is more preferably detected using a dry analytical element. In the method for analyzing a target nucleic acid fragment according to the present invention, the presence or abundance of the target nucleic acid fragment can be detected, or the nucleotide sequence of the target nucleic acid fragment can be detected. "Detection of the abundance" used herein refers to a concept including quantification of the target nucleic acid fragment. Specific examples of detection of the nucleotide sequence of the target nucleic acid fragment include detection of mutation or polymorphisms of the target nucleic acid fragment. Fig. 1 is a conceptual view illustrating an embodiment of the present invention.

A first preferred embodiment of the method for analyzing a target nucleic acid fragment according to the present invention is listed below.
(i) Detection of pyrophosphoric acid is carried out by using a dry analytical element for quantifying pyrophosphoric acid which comprises a reagent layer containing xanthosine or inosine, pyrophosphatase, purine nucleoside phosphorylase, xanthine oxidase, peroxidase, and a color developer.
(ii) Polymerase to be used is selected from the group consisting of DNA polymerase I, Klenow fragment of DNA polymerase I, Bst DNA polymerase, and reverse transcriptase.

Preferred embodiments of a method for analyzing a target nucleic acid fragment according to the second aspect of the present invention are listed below.
(i) Pyrophosphatase is used as an enzyme for converting pyrophosphoric acid.
(ii) Polymerase to be used is selected from the group consisting of DNA polymerase I, Klenow fragment of DNA polymerase I, Bst DNA polymerase, and reverse transcriptase.

The embodiments of the present invention will be described in more detail in the following.
(A) Target nucleic acid fragment: A target nucleic acid fragment to be analyzed in the present invention is a polynucleotide, at least a part of its nucleotide sequence being known, and can be a genomic DNA fragment isolated from all the organisms including animals, microorganisms, bacteria, and plants. Also, an RNA or DNA fragment which can be isolated from viruses and cDNA fragments which are synthesized using mRNA as a template, can be analyzed. Preferably, the target nucleic acid fragment is purified to the largest extent possible, and an extra ingredient other than a nucleic acid fragment is removed. For example, when genomic DNA fragments isolated from blood of an animal (e.g., human) or nucleic acid (DNA or RNA) fragments of infectious bacteria or virus existing in blood are analyzed, the cell membrane of leucocytes which has been destructed in the isolation process, hemoglobin which has been eluted from erythrocytes, and other general chemical substances in blood should be fully removed. In particular, hemoglobin inhibits the subsequent polymerase elongation reaction. Pyrophosphoric acid and phosphoric acid existing in blood as general biochemical substances are disturbing factors for accurate detection of pyrophosphoric acid generated by polymerase elongation reaction.
(B) Primer complementary with a target nucleic acid fragment: A primer complementary with a target nucleic acid fragment used in the present invention is an oligonucleotide having a nucleotide sequence being complementary with a target site, the nucleotide sequence of the target nucleic acid fragment being known. Hybridization of a primer complementary with the target nucleic acid fragment to a target site of the target nucleic acid fragment results in progress on polymerase elongation reaction starting from the 3' terminus of the primer and using the target nucleic acid as a template. Thus, whether or not the primer recognizes and specifically hybridizes to a target site of the target nucleic acid fragment is an important issue in the present invention. The number of nucleotides in the primer used in the present invention is preferably 5 to 60, and particularly preferably 15 to 40. If the number of nucleotides in the primer is too small, specificity with the target site of the target nucleic acid fragment is deteriorated and also a hybrid with the target nucleic acid fragment cannot be stably formed. When the number of nucleotides in the primer is too high, double-strands are disadvantageously formed due to hydrogen bonds between primers or between nucleotides in a primer. This also results in deterioration in specificity.
   When the existence of the target nucleic acid fragment is detected by the method according to the present invention, a plurality of primers complementary with each different site in the target nucleic acid fragment can be used. Thus, recognition of the target nucleic acid fragment in a plurality of sites results in improving the specificity in detecting the existence of the target nucleic acid fragment When a part of the target nucleic acid fragment is amplified (e.g., PCR), a plurality of primers can be designed in accordance with the amplification methods.
   When the nucleotide sequence of the target nucleic acid fragment is detected by the method according to the present invention, particularly when the occurrence of mutation or polymorphisms is detected, a primer is designed in accordance with a type of nucleotide corresponding to mutation or polymorphisms so as to contain a portion of mutation or polymorphisms of interest. Thus, the occurrence of mutation or polymorphisms of the target nucleic acid fragment causes a difference in the occurrence of hybridization of the primer to the target nucleic acid fragment, and the detection as difference in polymerase elongation reaction eventually becomes feasible. By setting a portion corresponding to mutation or polymorphisms around the 3' terminus of the primer, a difference in the recognition of the polymerase reaction site occurs, and this eventually enables the detection as difference in polymerase elongation reaction.
(C) Polymerase: When the target nucleic acid is DNA, polymerase used in the present invention is DNA polymerase which catalyzes complementary elongation reaction which starts from the double-strand portion formed by hybridization of the primer with the target nucleic acid fragment in its portion denatured into single-strand in the 5' → 3' direction by using deoxynucleoside triphosphate (dNTP) as the material and using the target nucleic acid fragment as a template. Specific examples of DNA polymerase used include DNA polymerase I, Klenow fragment of DNA polymerase I, and Bst DNA polymerase. DNA polymerase can be selected or combined depending on the purpose. For example, when a part of the target nucleic acid fragment is amplified (e.g., PCR), use of Taq DNA polymerase which is excellent in heat resistance, is effective. When a part of the target nucleic acid fragment is amplified by using the amplification method (loop-mediated isothermal amplification of DNA (the LAMP method)) described in "BIO INDUSTRY, Vol. 18, No. 2, 2001," use of Bst DNA polymerase is effective as strand displacement-type DNA polymerase which has no nuclease activity in the 5' → 3' direction and catalyzes the elongation reaction while allowing double-strand DNA to be released as single-strand DNA on the template. Use of DNA polymerase α, T4 DNA polymerase, and T7 DNA polymerase, which have hexokinase activity in the 3' → 5' direction in combination is also possible depending on the purpose.
   When a genomic nucleic acid of RNA viruses or mRNA is a target nucleic acid fragment, reverse transcriptase having reverse transcription activity can be used. Further, reverse transcriptase can be used in combination with Taq DNA polymerase.
(D) Polymerase elongation reaction: Polymerase elongation reaction in the present invention includes all the complementary elongation reactions of nucleic acids which proceed by starting from the 3' terminus of a primer complementary with the target nucleic acid fragment as described in (B) above which was specifically hybridized with a part of the portion denatured into a single-strand of the target nucleic acid fragment as described in (A), using deoxynucleoside triphosphate (dNTP) as material, using a polymerase as described in (C) above as a catalyst, and using a target nucleic acid fragment as a template. This complementary nucleic acid elongation reaction indicates that a continuous elongation reaction occurs at least twice (corresponding to 2 nucleotides).
   Examples of representative polymerase elongation reactions and amplification reactions of a subject site of the target nucleic acid fragment involving polymerase elongation reactions are shown below. The simplest case is that only one polymerase elongation reaction in the 5' → 3' direction is carried out using the target nucleic acid fragment as a template. This polymerase elongation reaction can be carried out under isothermal conditions. In this case, the amount of pyrophosphoric acid generated as a result of polymerase elongation reaction is in proportion to the initial amount of the target nucleic acid fragment. Specifically, it is a suitable method for quantitatively detecting the existence of the target nucleic acid fragment.
   When the amount of the target nucleic acid is small, a target site of the target nucleic acid is preferably amplified by any means utilizing polymerase elongation reaction. In the amplification of the target nucleic acid, various methods which have been heretofore developed, can be used. The most general and spread method for amplifying the target nucleic acid is polymerase chain reaction (PCR). PCR is a method of amplifying a target portion of the target nucleic acid fragment by repeating periodical processes of denaturing (a step of denaturing a nucleic acid fragment from double-strand to single-strand) → annealing (a step of hybridizing a primer to a nucleic acid fragment denatured into single-strand) → polymerase (Taq DNA polymerase) elongation reaction → denaturing, by periodically controlling the increase and decrease in temperature of the reaction solution. Finally, the target site of the target nucleic acid fragment can be amplified 1,000,000 times as compared to the initial amount. Thus, the amount of accumulated pyrophosphoric acid generated by polymerase elongation reaction in the amplification process in PCR becomes large, and thereby the detection becomes easy.
   A cyclic assay method using exonuclease described in Laid-Open Japanese Patent Publication No. 5-130870 is a method for amplifying a target site of the target nucleic acid fragment utilizing polymerase elongation. In this method, a primer is decomposed from a reverse direction by performing polymerase elongation reaction starting from a primer specifically hybridized with a target site of the target nucleic acid fragment, and allowing 5' → 3' exonuclease to act. In place of the decomposed primer, a new primer is hybridized, and the elongation reaction by DNA polymerase proceeds again. This elongation reaction by polymerase and the decomposition reaction by exonuclease for removing the previously elongated strand are successively and periodically repeated. The elongation reaction by polymerase and the decomposition reaction by exonuclease can be carried out under isothermal conditions. The amount of accumulated pyrophosphoric acid generated by the polymerase elongation reaction repeated in this cyclic assay method becomes large, and the detection becomes easy.
   The LAMP method is a recently developed method for amplifying a target site of the target nucleic acid fragment. This method is carried out by using at least 4 types of primers, which complimentarily recognize at least 6 specific sites of the target nucleic acid fragment, and strand displacement-type Bst DNA polymerase, which has no nuclease activity in the 5' → 3' direction and which catalyzes the elongation reaction while allowing the double-strand DNA on the template to be released as single-strand DNA. In this method, a target site of the target nucleic acid fragment is amplified as a special structure under isothermal conditions. The amplification efficiency of the LAMP method is high, and the amount of accumulated pyrophosphoric acid generated by the polymerase elongation reaction is very large, and the detection becomes easy.
   When the target nucleic acid fragment is a RNA fragment, the elongation reaction can be carried out by using reverse transcriptase having reverse transcription activity and using the RNA strand as a template. Further, RT-PCR can be utilized where reverse transcriptase is used in combination with Taq DNA polymerase, and the reverse transcription (RT) reaction is carried out, followed by PCR. Detection of pyrophosphoric acid generated in the RT reaction or RT-PCR reaction enables the detection of the existence of the RNA fragment of the target nucleic acid fragment. This method is effective when the existence of RNA viruses is detected.
(E) Detection of pyrophosphoric acid (PPi): A method represented by formula 1 has been heretofore known as a method for detecting pyrophosphoric acid (PPi). In this method, pyrophosphoric acid (PPi) is converted into adenosinetriphosphate (ATP) with the aid of sulfurylase, and the luminescence generated when adenosinetriphosphate acts on luciferin with the aid of luciferase is detected. Thus, an apparatus capable of measuring luminescence is required for detecting pyrophosphoric acid (PPi) by this method. 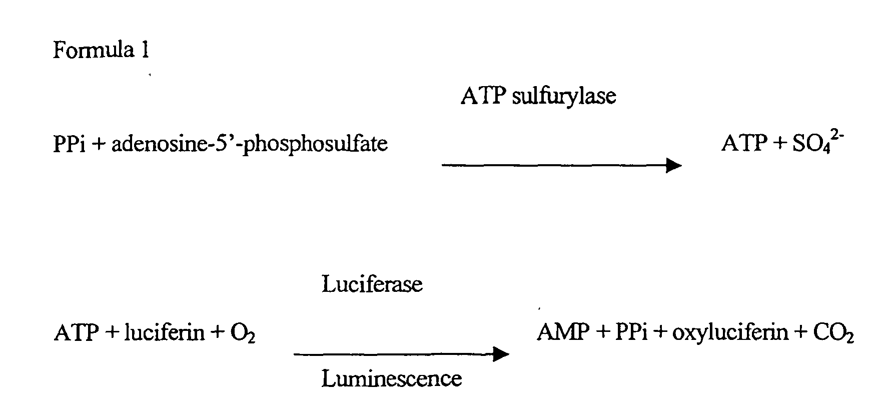
   A method for detecting pyrophosphoric acid suitable for the present invention is a method represented by formula 2 or 3. In the method represented by formula 2 or 3, pyrophosphoric acid (PPi) is converted into inorganic phosphate (Pi) with the aid of pyrophosphatase, inorganic phosphate (Pi) is reacted with xanthosine or inosine with the aid of purine nucleoside phosphorylase (PNP), the resulting xanthine or hypoxanthine is oxidized with the aid of xanthine oxidase (XOD) to generate uric acid, and a color developer (a dye precursor) is allowed to develop color with the aid of peroxidase (POD) using hydrogen peroxide (H₂O₂) generated in the oxidation process, followed by colorimetry. In the method represented by formula 2 or 3, the result can be detected by colorimetry and, thus, pyrophosphoric acid (PPi) can be detected visually or using a simple colorimetric measuring apparatus. 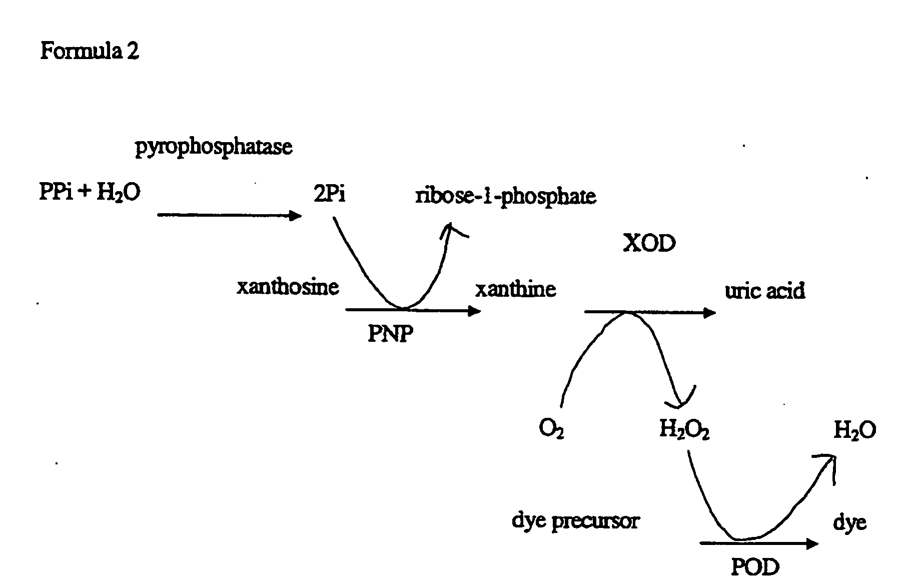 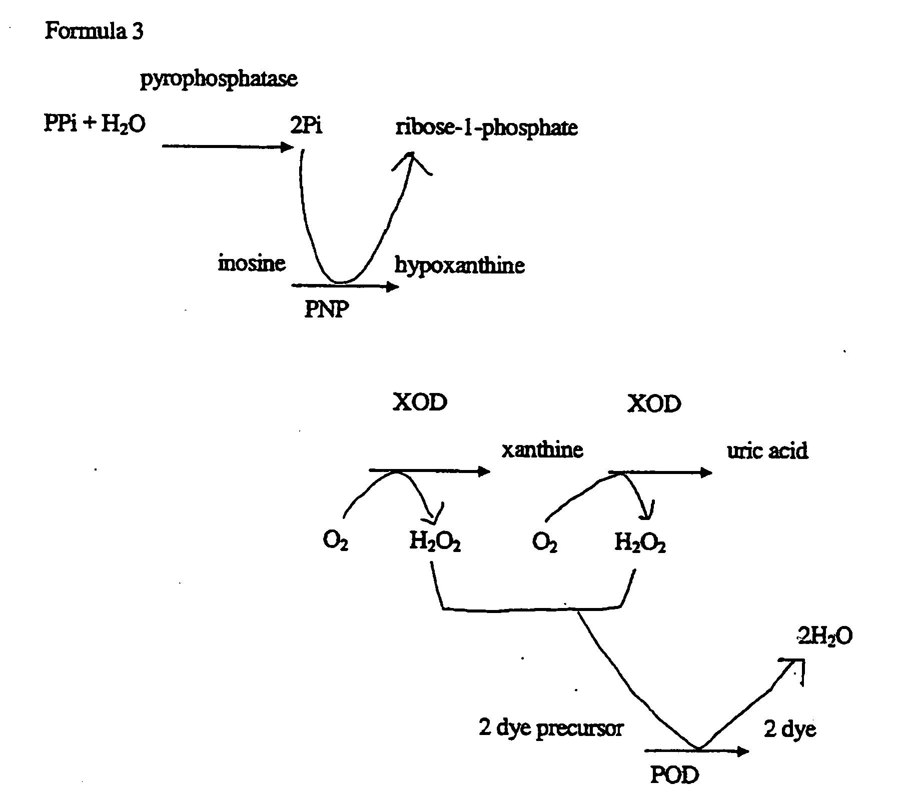
   Commercially available pyrophosphatase (EC3, 6, 1, 1), purine nucleoside phosphorylase (PNP, EC2. 4. 2. 1), xanthine oxidase (XOD, EC1. 2. 3. 2), and peroxidase (POD, EC1. 11. 1. 7) can be used. A color developer (i.e., a dye precursor) may be any one as long as it can generate a dye with hydrogen peroxide and peroxidase (POD), and examples thereof which can be used herein include: a composition which generates a dye upon oxidation of the leuco dye (e.g., triarylimidazole leuco dye described in U.S.Patent. No. 4,089,747 and the like, diarylimidazole leuco dye described in Laid-Open Japanese Patent Publication No. 59-193352 (EP 0122641A)); and a composition (e.g., 4-aminoantipyrines and phenols or naphthols) containing a compound generating a dye by coupling with another compound upon oxidation.
(F) Dry analytical element: A dry analytical element which can be used in the present invention is an analytical element which comprises a single or a plurality of functional layers, wherein at least one layer (or a plurality of layers) comprises a detection reagent, and a dye generated by reaction in the layer is subjected to a quantification by colorimetry by reflected light or transmitted light from outside the analytical element.
   In order to perform a quantitative analysis using such a dry analytical element, a given amount of liquid sample is spotted onto the surface of a developing layer. The liquid sample spread on the developing layer reaches the reagent layer and reacts with the reagent thereon and develops a color. After spotting, the dry analytical element is maintained for a suitable period of time at a given temperature (for incubation) and a color developing reaction is allowed to thoroughly proceed. Thereafter, the reagent layer is irradiated with an illuminating light from, for example, a transparent support side, the amount of reflected light in a specific wavelength region is measured to determine the optical density of reflection, and a quantitative analysis is carried out based on the previously determined calibration curve.
   Since a dry analytical element is stored and kept in a dry state before detection, it is not necessary that a reagent is prepared for each use. As the stability of the reagent is generally higher in a dry state, it is better than a so-called wet process in terms of simplicity and swiftness since the wet process requires the preparation of the reagent solution for each use. It is also excellent as an examination method because highly accurate examination can be swiftly carried out with a very small amount of liquid sample.
(G) Dry analytical element for quantifying pyrophosphoric acid: A dry analytical element for quantifying pyrophosphoric acid which can be used in the present invention can have a layer construction which is similar to that of various known dry analytical elements. The dry analytical element may consist of multiple layers which contain, in addition to a reagent for performing the reaction represented by formula 2 or 3 according to item (E) above (detection of pyrophosphoric acid (PPi)), a support, a developing layer, a detection layer, a light-shielding layer, an adhesive layer, a water-absorption layer, an undercoating layer, and other layers. Examples of such dry analytical elements include those disclosed in the specifications of Laid-Open Japanese Patent Publication No. 49-53888 (U.S. Patent. No. 3,992,158), Laid-Open Japanese Patent Publication No. 51-40191 (U.S. Patent. No. 4,042,335), Laid-Open Japanese Patent Publication No. 55-164356 (U.S.Patent No. 4,292,272), and Laid-Open Japanese Patent Publication No. 61-4959 (EPC Publication No. 0166365A).

Examples of the dry analytical element to be used in the present invention include a dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus.

In this dry analytical element for quantitative assay of pyrophosphate, pyrophosphoric acid (PPi) can enzymatically be converted into inorganic phosphorus (Pi) using pyrophosphatase as described above. The subsequent process, that is the color reaction depending on the amount of inorganic phosphorus (Pi), can be performed using the "quantitative assay method of inorganic phosphorus" (and combinations of individual reactions used therefor), described hereinafter, which is known in the field of biochemical inspection.

It is noted that when representing "inorganic phosphorus," both the expressions "Pi" and "HPO₄²⁻, H₂PO₄¹⁻" are used for phosphoric acid (phosphate ion). Although the expression "Pi" is used in the examples of reactions described below, the expression "HPO₄²⁻" may be used for the same reaction formula.

As the quantitative assay method of inorganic phosphorus, an enzyme method and a phosphomolybdate method are known. Hereinafter, this enzyme method and phosphomolybdate method will be described as the quantitative assay method of inorganic phosphorus.

### A. Enzyme method

Depending on the enzyme to be used for the final color reaction during a series of reactions for Pi quantitative detection, the following methods for quantitative assay are available: using peroxidase (POD); or using glucose-6-phosphate dehydrogenase (G6PDH), respectively. Hereinafter, examples of these methods are described.

### (1) Example of the method using peroxidase (POD)

### (1-1)

Inorganic phosphorus (Pi) is allowed to react with inosine by purine nucleoside phosphorylase (PNP), and the resultant hypoxanthine is oxidized by xanthine oxidase (XOD) to produce uric acid. During this oxidation process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye, which is colorimetrically assessed.

### (1-2)

Pyruvic acid is oxidized by pyruvic oxidase (POP) in the presence of inorganic phosphorus (Pi), cocarboxylase (TPP), flavin adenine dinucleotide (FAD) and Mg²⁺ to produce acetyl acetate. During this oxidation process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye which is colorimetrically assessed, in the same manner as described in (1-1).

It is noted that the final color reaction for each of the above processes (1-1) and (1-2) can be performed by a "Trinder reagent" which is known as a detection reagent for hydrogen peroxide. In this reaction, phenols function as "hydrogen donors." Phenols to be used as "hydrogen donors" are classical, and now various modified "hydrogen donors" are used. Examples of these hydrogen donors include N-ethyl-N-sulfopropyl-m-anilidine, N-ethyl-N-sulfopropylaniline, N-ethyl-N-sulfopropyl-3,5-dimethoxyaniline, N-sulfopropyl-3,5-dimethoxyaniline, N-ethyl-N-sulfopropyl-3,5-dimethylaniline, N-ethyl-N-sulfopropyl-m-toluidine, N-ethyl-N-(2-hydroxy-3-sulfopropyl)-m-anilidine , N-ethyl-N-(2-hydroxy-3-sulfopropyl)aniline, N-ethyl-N-(2-hydroxy-3-sulfopropyl)-3,5-dimethoxyaniline, N-(2-hydroxy-3-sulfopropyl)-3,5-dimethoxyaniline, N-ethyl-N-(2-hydroxy-3-sulfopropyl)-3,5-dimethylaniline, N-ethyl-N-(2-hydroxy-3-sulfopropyl)-m-toluidine, and N-sulfopropylaniline.

### (2) Example of a method using glucose-6-phosphate dehydrogenase (G6PDH)

### (2-1)

Inorganic phosphorus (Pi) is reacted with glycogen and phosphorylase to produce glucose-1-phosphate (G-1-P). The produced glucose-1-phosphate is converted into glucose-6-phosphate (G-6-P) with phosphoglucomutase (PGM). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### (2-2)

Inorganic phosphorus (Pi) is reacted with maltose and maltose phosphorylase (MP) to produce glucose-1-phosphate (G-1-P). Thereafter, the produced glucose-1-phosphate is converted into glucose-6-phosphate (G-6-P) with phosphoglucomutase (PGM) in the same manner as described in (2-1). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### B. Phosphomolybdate method

There are two phosphomolybdate methods. One is a direct method wherein "Phosphomolybdates (H₃[POMo₁₂O₃₆])" prepared by complexing inorganic phosphorus (phosphate) and aqueous molybdate ions under acidic condition are directly quantified. The other is a reduction method wherein further to the above direct method, Mo(IV) is reduced to Mo(III) by a reducing agent and molybudenum blue (Mo(III)) is quantified. Examples of the aqueous molybdate ions include aluminum molybdate, cadmium molybdate, calcium molybdate, barium molybdate, lithium molybdate, potassium molybdate, sodium molybdate, and ammonium molybdate. Representative examples of the reducing agents to be used in the reduction method include 1-amino-2-naphthol-4-sulfonic acid, ammonium ferrous sulfate, ferrous chloride, stannous chloride-hydrazine, *p*-methylaminophenol sulfate, N,N-dimethyl-phenylenediamine, ascorbic acid, and malachite green.

When a light-transmissive and water-impervious support is used, the dry analytical element can be practically constructed as below. However, the scope of the present invention is not limited to these.
(1) One having a reagent layer on the support.
(2) One having a detection layer and a reagent layer in that order on the support.
(3) One having a detection layer, a light reflection layer, and a reagent layer in that order on the support.
(4) One having a second reagent layer, a light reflection layer, and a first reagent layer in that order on the support.
(5) One having a detection layer, a second reagent layer, a light reflection layer, and a first reagent layer in that order on the support.

In (1) to (3) above, the reagent layer may be constituted by a plurality of different layers. For example, a first reagent layer may contain enzyme pyrophosphatase which is required in the pyrophosphatase reaction represented by formula 2 or 3, and the substrate xanthosine or the substrate inosine and the enzyme PNP which are required in the PNP reaction, a second reagent layer may contain enzyme XOD which is required in the XOD reaction represented by formula 2 or 3, and a third reagent layer may contain enzyme POD which is required in the POD reaction represented by formula 2 or 3, and a coloring dye (dye precursor). Alternatively, two reagent layers are provided. On the first reagent layer, the pyrophosphatase reaction and the PNP reaction may be proceeded, and the XOD reaction and the POD reaction may be proceeded on the second reagent layer. Alternatively, the pyrophosphatase reaction, the PNP reaction and the XOD reaction may be proceeded on the first reagent layer, and the POD reaction may be proceeded on the second reagent layer.

A water absorption layer may be provided between a support and a reagent layer or detection layer. A filter layer may be provided between each layer. A developing layer may be provided on the reagent layer and an adhesive layer may be provided therebetween.

Any of light-nontransmissive (opaque), light-semitransmissive (translucent), or light-transmissive (transparent) support can be used. In general, a light-transmissive and water-impervious support is preferred. Preferable materials for a light-transmissive and water-impervious support are polyethylene terephthalate or polystyrene. In order to firmly adhere a hydrophilic layer, an undercoating layer is generally provided or hydrophilization is carried out.

When a porous layer is used as a reagent layer, the porous medium may be a fibrous or nonfibrous substance. Fibrous substances used herein include, for example, filter paper, non-woven fabric, textile fabric (e.g. plain-woven fabric), knitted fabric (e.g., tricot knitted fabric), and glass fiber filter paper. Nonfibrous substances may be any of a membrane filter comprising cellulose acetate etc., described in Laid-Open Japanese Patent Publication No. 49-53888 and the like, or a particulate structure having mutually interconnected spaces comprising fine particles of inorganic substances or organic substances described in, for example, Laid-Open Japanese Patent Publication No. 49-53888, Laid-Open Japanese Patent Publication No. 55-90859 (U.S. Patent. No. 4,258,001), and Laid-Open Japanese Patent Publication No. 58-70163 (U.S. Patent. No. 4,486,537). A partially-adhered laminate which comprises a plurality of porous layers described in, for example, Laid-Open Japanese Patent Publication No. 61-4959 (EP Publication 0166365A), Laid-Open Japanese Patent Publication No. 62-116258, Laid-Open Japanese Patent Publication No. 62-138756 (EP Publication 0226465A), Laid-Open Japanese Patent Publication No. 62-138757 (EP Publication 0226465A), and Laid-Open Japanese Patent Publication No. 62-138758 (EP Publication 0226465A), is also preferred.

A porous layer may be a developing layer having so-called measuring action, which spreads liquid in an area substantially in proportion to the amount of the liquid to be supplied. Preferably, a developing layer is textile fabric, knitted fabric, and the like. Textile fabrics and the like may be subjected to glow discharge treatment as described in Laid-Open Japanese Patent Publication No. 57-66359. A developing layer may comprise hydrophilic polymers or surfactants as described in Laid-Open Japanese Patent Publication No. 60-222770 (EP 0162301A), Laid-Open Japanese Patent Publication No. 63-219397 (German Publication DE 3717913A), Laid-Open Japanese Patent Publication No. 63-112999 (DE 3717913A), and Laid-Open Japanese Patent Publication No. 62-182652 (DE 3717913A) in order to regulate a developing area, a developing speed and the like.

For example, a method is useful in which the reagent of the present invention is previously impregnated into or coated on a porous membrane etc., comprising paper, fabric or polymer, followed by adhesion onto another water-pervious layer provided on a support (e.g., a detection layer) by the method as described in Laid-Open Japanese Patent Publication No. 55-1645356.

The thickness of the reagent layer thus prepared is not particularly limited. When it is provided as a coating layer, the thickness is suitably in the range of about 1 µm to 50 µm, preferably in the range of 2 µm to 30 µm. When the reagent layer is provided by a method other than coating, such as lamination, the thickness can be significantly varied in the range of several tens of to several hundred µm.

When a reagent layer is constituted by a water-pervious layer of hydrophilic polymer binders, examples of hydrophilic polymers which can be used include: gelatin and a derivative thereof (e.g., phthalated gelatin); a cellulose derivative (e.g., hydroxyethyl cellulose); agarose, sodium arginate; an acrylamide copolymer or a methacrylamide copolymer (e.g., a copolymer of acrylamide or methacrylamide and various vinyl monomers); polyhydroxyethyl methacrylate; polyvinyl alcohol; polyvinyl pyrrolidone; sodium polyacrylate; and a copolymer of acrylic acid and various vinyl monomers.

A reagent layer composed of hydrophilic polymer binders can be provided by coating an aqueous solution or water dispersion containing the reagent composition of the present invention and hydrophilic polymers on the support or another layer such as a detection layer followed by drying the coating in accordance with the methods described in the specifications of Japanese Patent Examined Publication No. 53-21677 (U.S. Patent No. 3,992,158), Laid-Open Japanese Patent Publication No. 55-164356 (U.S. Patent. No. 4,292,272), Laid-Open Japanese Patent Publication No. 54-101398 (U.S. Patent. No. 4,132,528) and the like. The thickness of the reagent layer comprising hydrophilic polymers as binders is about 2 µm to about 50 µm, preferably about 4 µm to about 30 µm on a dry basis, and the coverage is about 2 g/m² to about 50 g/m², preferably about 4 g/m² to about 30 g/m².

The reagent layer can further comprise an enzyme activator, a coenzyme, a surfactant, a pH buffer composition, an impalpable powder, an antioxidant, and various additives comprising organic or inorganic substances in addition to the reagent composition represented by formula 2 or 3 in order to improve coating properties and other various properties of diffusible compounds such as diffusibility, reactivity, and storage properties. Examples of buffers which can be contained in the reagent layer include pH buffer systems described in "Kagaku Binran Kiso (Handbook on Chemistry, Basic)," The Chemical Society of Japan (ed.), Maruzen Co., Ltd. (1996), p.1312-1320, "Data for Biochemical Research, Second Edition, R M. C. Dawson et al. (2nd ed.), Oxford at the Clarendon Press (1969), p. 476-508, "Biochemistry" 5, p. 467-477 (1966), and "Analytical Biochemistry" 104, p. 300-310 (1980). Specific examples of pH buffer systems include a buffer containing borate; a buffer containing citric acid or citrate; a buffer containing glycine, a buffer containing bicine; a buffer containing HEPES; and Good's buffers such as a buffer containing MES. A buffer containing phosphate cannot be used for a dry analytical element for detecting pyrophosphoric acid.

The dry analytical element for quantifying pyrophosphoric acid which can be used in the present invention can be prepared in accordance with a known method disclosed in the above-described various patent specifications. The dry analytical element for quantifying pyrophosphoric acid is cut into small fragments, such as, an about 5 mm to about 30 mm-square or a circle having substantially the same size, accommodated in the slide frame described in, for example, Japanese Patent Examined Publication No. 57-283331 (U.S. Patent. No. 4,169,751), Laid-Open Japanese Utility Model Publication No. 56-142454 (U.S. Patent. No. 4,387,990), Laid-Open Japanese Patent Publication No. 57-63452,Laid-Open Japanese Utility Model Publication No. 58-32350, and Laid-Open Japanese Patent Publication No. 58-501144 (International Publication WO 83/00391), and used as slides for chemical analysis. This is preferable from the viewpoints of production, packaging, transportation, storage, measuring operation, and the like. Depending on its intended use, the analytical element can be accommodated as a long tape in a cassette or magazine, as small pieces accommodated in a container having an opening, as small pieces applied onto or accommodated in an open card, or as small pieces cut to be used in that state.

The dry analytical element for quantifying pyrophosphoric acid which can be used in the present invention can quantitatively detect pyrophosphoric acid which is a test substance in a liquid sample, by operations similar to that described in the above-described patent specifications and the like. For example, about 2 µL to about 30 µL, preferably 4 µL to 15 µL of aqueous liquid sample solution is spotted on the reagent layer. The spotted analytical element is incubated at a constant temperature of about 20°C to about 45°C, preferably about 30°C to about 40°C for 1 to 10 minutes. Coloring or discoloration in the analytical element is measured by the reflection from the light-transmissive support side, and the amount of pyrophosphoric acid in the specimen can be determined based on the principle of colorimetry using the previously prepared calibration curve. Quantitative analysis can be carried out with high accuracy by keeping the amount of liquid sample to be spotted, the incubation time, and the temperate at constant levels.

Quantitative analysis can be carried out with high accuracy in a very simple operation using chemical analyzers described in, for example, Laid-Open Japanese Patent Publication No. 60-125543, Laid-Open Japanese Patent Publication No. 60-220862, Laid-Open Japanese Patent Publication No. 61-294367, and Laid-Open Japanese Patent Publication No. 58-161867 (U.S. Patent. No. 4,424,191). A semiquantitative measurement may be carried out by visually judging the level of coloring depending on the purpose and accuracy needed.

Since the dry analytical element for quantifying pyrophosphoric acid which can be used in the present invention is stored and kept in a dry state before analysis, it is not necessary that a reagent is prepared for each use, and the stability of the reagents is generally higher in a dry state. Thus, in terms of simplicity and swiftness, it is better than a so-called wet process, which requires the preparation of the reagent solution for each use. It is also excellent as an examination method because highly accurate examination can be swiftly carried out with a very small amount of liquid sample.

The dry analytical element for quantifying inorganic phosphorus which can be used in the second aspect of the present invention can be prepared by removing pyrophosphatase from the reagent layer in the aforementioned dry analytical element for quantifying pyrophosphoric acid The dry analytical element described in Laid-Open Japanese Patent Publication No. 7-197 can also be used. The dry analytical element for quantifying inorganic phosphorus is similar to the aforementioned dry analytical element for quantifying pyrophosphoric acid in terms of its layer construction, method of production, and method of application, with the exception that the reagent layer does not comprise pyrophosphatase.
(H) Kit: The analysis of the target nucleic acid according to the present invention can be analyzed using a kit comprising at least one primer complementary with a part of the target nucleic acid fragment to be analyzed, at least one deoxynucleoside triphosphate (dNTP), at least one polymerase, and a dry analytical element for quantifying pyrophosphoric acid.

The form of the kit may be a cartridge comprising: an opening capable of supplying a liquid containing the target nucleic acid fragment, at least a part of its nucleotide sequence being known; at least one primer complementary with a part of the target nucleic acid fragment; at least one deoxynucleoside triphosphate (dNTP), at least one reaction cell unit capable of retaining at least one polymerase; a detection unit capable of retaining a dry analytical element for quantifying pyrophosphoric acid; and a canaliculus or groove capable of connecting the opening, the reaction cell unit, and the detection unit and transferring liquid among them.

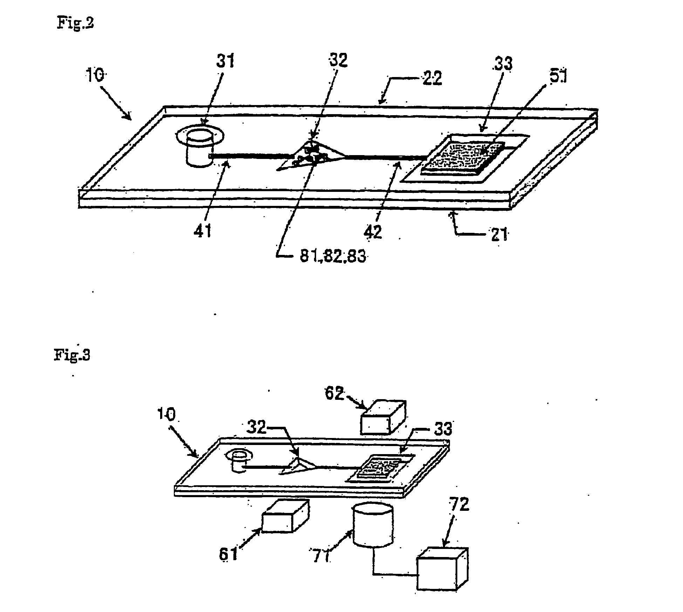

The cartridge disclosed in U.S. Patent No. 5,919,711 and the like can be used as such a cartridge. An embodiment of a kit according to the present invention in the form of a cartridge is shown in Fig. 2. In the kit 10, a sample liquid containing the target nucleic acid can be supplied from an opening 31. The opening 31 is connected to a reaction cell 32 through a canaliculus 41. The reaction cell 32 contains in advance at least one primer 81 complementary with a part of the target nucleic acid fragment, at least one deoxynucleoside triphosphate (dNTP) 82, and at least one polymerase 83. The reaction cell 32 is further connected to a detection unit 33 through canaliculus 42. The detection unit 33 contains in advance the dry analytical element 51. The sample solution, in which the polymerase elongation reaction has proceeded in reaction cell 32, is transferred through canaliculus 42, supplied on the dry analytical element 51 for quantifying pyrophosphoric acid in the detection unit 33, and detects pyrophosphoric acid generated by polymerase elongation reaction. In the kit 10, liquid transference between the opening 31 and the reaction cell 32 and between the reaction cell 32 and the detection unit 33 can be carried out by centrifuge force, electrophoresis, electroosmosis, or the like. Preferably, the reaction cell 32, the canaliculuses 41 and 42, and the detection unit 33 are hermetically sealed with a substrate 21 and a lid 22.

When the kit 10 in the form of cartridge as shown in Fig. 2 is used, as shown in Fig. 3, an apparatus which comprises temperature control units 61 and 62 of the reaction cell 32 and the detection unit 33 and the detection units 71 and 72 capable of detecting coloring or color change in the dry analytical element 51 for quantifying pyrophosphoric acid by reflection light, is preferably used in combination.

The kit in the form of cartridge which can be used in the present invention is not limited to those shown in Fig. 2. Reagents required in polymerase elongation reaction may be respectively retained in separate spaces. In that case, each reagent may be transferred to a reaction cell at the time of reaction. There may be a plurality of reaction cells.

When pyrophosphoric acid generated by polymerase elongation reaction is detected by enzymatically converting pyrophosphoric acid into inorganic phosphoric acid, followed by the use of dry analytical element for quantifying inorganic phosphorus, at least one primer complementary with a part of the target nucleic acid fragment, at least one deoxynucleoside triphosphate (dNTP), and at least one polymerase are previously retained in the first reaction cell, and polymerase elongation reaction is carried out in the first reaction cell. Subsequently, the reaction solution generated in the first reaction cell is transferred to the second reaction cell, which is connected to the first reaction cell through a canaliculus and which already contains pyrophosphatase, pyrophosphoric acid generated in polymerase elongation reaction in the first reaction cell is converted into inorganic phosphoric acid in the second reaction cell. The reaction solution in the second reaction cell is then transferred to the detection unit, which is connected to the second reaction cell through a canaliculus and previously retains a dry analytical element for quantifying inorganic phosphorus, thereby detecting inorganic phosphorus.

A set of "opening - canaliculus - reaction cell - canaliculus - detection unit" is arranged in parallel on one cartridge, or plural sets thereof can be arranged in concentric circles in the radius direction. In this case, for example, the nucleotide sequence of at least one primer complementary with a part of the target nucleic acid fragment retained in the reaction cell can be modified in accordance with the type of the targeted nucleic acid to provide a kit capable of simultaneously detecting a plurality of target nucleic acids.

The present invention is described in more detail with reference to the following examples. However, the technical scope of the present invention is not limited by these examples.

### Examples

### Example 1: Detection of the SRY gene-associated site on the short arm of the Y chromosome using a dry analytical element for quantifying pyrophosphoric acid

### (1) Preparation of the dry analytical element for quantifying pyrophosphoric acid

An aqueous solution having composition (a) shown in Table 1 was coated at the following coverage on a colorless transparent polyethylene terephthalate (PET) smooth film sheet (support) having a gelatin undercoating layer (thickness of 180 µm). The coating was then dried to provide a reagent layer.

**Table 1**

| Composition (a) of an aqueous solution for the reagent layer | |
|---|---|
| Gelatin | 18.8 g/m² |
| p-Nonylphenoxy polyxydol (glycidol unit: containing 10 units on average) (C₉H₁₉-Ph-O-(CH₂CH(OH)-CH₂-O)₁₀H) | 1.5 g/m² |
| Xanthosine | 1.96 g/m² |
| Peroxidase | 15,000 IU/m² |
| Xanthine oxidase | 13,600 IU/m² |
| Purine nucleoside phosphorylase | 3,400 IU/m² |
| Pyrophosphatase | 15,000 IU/m² |
| Leuco dye (2-(3,5-dimethoxy-4-hydroxydiphenyl)-4- phenethyl-5-(4-dimethylaminophenyl)imidazole | 0.28 g/m² |
| Water (pH was adjusted to 6.8 with a diluted NaOH solution) | 136 g/m² |

On this reagent layer, an aqueous solution for an adhesive layer having composition (b) shown in Table 2 below was coated at the following coverage. The coating was then dried to provide an adhesive layer.

**Table 2**

| Composition (b) of an aqueous solution for the adhesive layer | |
|---|---|
| Gelatin | 3.1 g/m² |
| p-Nonylphenoxy polyxydol (glycidol unit: containing 10 units on average) (C₉H₁₉-Ph-O-(CH₂CH(OH)-CH₂-O)₁₀H) | 0.25 g/m² |
| Water | 59 g/m² |

Subsequently, water was supplied on the adhesive layer over its whole surface at 30 g/m² to allow the gelatin layer to swell. A broad textile fabric made of genuine polyester was laminated thereon by applying slight pressure in a substantially even manner to provide a porous developing layer.

An aqueous solution having composition (c) shown in Table 3 below was then substantially evenly coated on the developing layer at the following coverage. The coating was then dried to prepare a dry analytical element for quantifying pyrophosphoric acid.

**Table 3**

| Composition (c) of an aqueous coating solution for the developing layer | |
|---|---|
| HEPES | 2.1 g/m² |
| Hydroxypropyl methyl cellulose (methoxy group 19 to 24%, hydroxypropoxy group 4 to 12%, viscosity of 2% aqueous solution at 20°C: 80 to 120 cps) | 0.9 g/m² |
| Surfactant (polyoxyethylene octyl phenyl ether) (C₈H₁₇-Ph-(O-CH_{2C}H₂-)₄₀-OH) | 2.7 g/m² |
| Titanium dioxide (rutile type) | 4.2 g/m² |
| Water (pH was adjusted to 7.5 with a diluted NaOH solution) | 90.0 g/m² |

### (2) Preparation of the sample solution of the target nucleic acid fragment

Blood specimens were collected from 1 male and 1 female, and a genomic nucleic acid fragment was extracted and purified therefrom using a commercially available kit for extracting and purifying nucleic acids (QIAGEN, QIAamp DNA Blood Mini Kit). The genomic nucleic acid fragment was then collected into 1 mL of purified distilled water, thereby preparing a sample solution of the target nucleic acid fragment.

### (3) Preparation of the primer

A primer was synthesized as a set of oligonucleotide primers (primer 1, primer 2) having a nucleotide sequence designed to specifically recognize the SRY gene on the short arm of the Y chromosome.

### <Nucleotide sequence of the primers>

Primer 1: 5'-GATCAGCAAGGAGCTGGGATACACGTG-3' (SEQ ID NO: 1)
Primer 2: 5'-CTGTAGCTTCCCGTTGCGGTG-3' (SEQ ID NO: 2)

### (4) Amplification of the target nucleotide acid fragment by polymerase elongation reaction

The target nucleic acid fragment was amplified by PCR using a reaction solution having the composition below. PCR was carried out by repeating 30 cycles of denaturing at 94°C for 30 seconds, annealing at 65°C for 30 seconds, and polymerase elongation reaction at 72°C for 1 minute.

### <Composition of the reaction solution>

| | |
|---|---|
| Purified water | 36.5 µL |
| 10x PCR buffer | 5 µL |
| 2.5 mM dNTP | 4 µL |
| Taq FP (manufactured by NIPPON GENE CO., LTD.) | 0.5 µL |
| 20 µM primer | 2 µL |
| 30 ng/µL sample solution of the target nucleic acid fragment | 2 µL |

### (5) Detection of pyrophosphoric acid using an analytical element for quantifying pyrophosphoric acid

In the amplification of the target nucleic acid fragment by polymerase elongation reaction according to (4) above, 10 µl L of the reaction solutions obtained was spotted onto the dry analytical element for quantifying pyrophosphoric acid prepared in (1) above for each of the following cases: a sample solution containing no target nucleic acid fragment (control); a sample solution of target nucleic acid prepared from blood collected from a male (sample M); and a sample solution of target nucleic acid prepared from blood collected from a female (sample F). The dry analytical element for quantifying pyrophosphoric acid was incubated at 37°C for 5 minutes, and the reflection density (OD_{R}) was then measured at a wavelength of 650 nm from the support side. As a result, it was 0.287, 1.143, and 0.281 for a control, sample M, and sample F, respectively.

Example 1 demonstrates that the SRY gene-associated site on the short arm of the Y chromosome existing peculiarly in males can be specifically detected. From the result of Example 1, it can be understood that the existence of the target nucleic acid fragment can be detected by the method according to the present invention in which pyrophosphoric acid generated as the polymerase elongation progresses, is detected using a dry analytical element for quantifying pyrophosphoric acid.

### Example 2: Detection of single nucleotide polymorphisms (SNPs) of an aldehyde dehydrogenase gene (ALDH2 gene)-associated site using dry analytical element for quantifying pyrophosphoric acid

### (1) Preparation of the sample solution of the target nucleic acid fragment

Based on each of the blood specimens collected from each of the subjects, who are known to be either in the active form of ALDH2 or in the inactive form of ALDH2 by nucleotide sequencing because of differences in a specific type of nucleotide in the ALDH2 gene-associated site, sample solutions of the target nucleic acid fragment were prepared as the sample active form of ALDH2 and the sample inactive form of ALDH2, respectively, in the same manner as described in (2) in Example 1.

### (2) Design of the primer

A primer was synthesized as a set of oligonucleotide primers: an oligonucleotide primer (primer 1) having a nucleotide sequence designed as a primer specific to the ALDH2 active nucleotide sequence for a specific portion determining the ALDH2 activity in the ALDH2 gene-associated site on chromosome 12; and an oligonucleotide primer (primer 2) having a nucleotide sequence designed as a primer specific to the nucleotide sequence downstream of the specific site.

### <Nucleotide sequence of the primers>

Primer 1: 5'-CAGGCATACACTGAAGTGAAAACTG-3' (SEQ ID NO: 3) (if the nucleotide sequence of the underlined GAA becomes AAA, it becomes an inactive form of ALDH2
Primer 2: 5'-AGGTCCTGAACTTCCAGCAG-3' (SEQ ID NO: 4)

### (3) Detection of pyrophosphoric acid using an analytical element for quantifying pyrophosphoric acid

The analytical element for quantifying pyrophosphoric acid was prepared in the same manner as described in (1) in Example 1, the target nucleic acid fragment was amplified (PCR) by polymerase elongation reaction in the same manner as described in (4) in Example 1, and the reaction solution was assayed after the polymerase elongation reaction using the dry analytical element for analyzing pyrophosphoric acid in the same manner as described in (5) in Example 1. As a result of the measurement of the reflection density (ORR), it was 0.256,1.003, and 0.262 for the control, the sample of active form of ALDH2, and the sample of inactive form of ALDH2, respectively.

Example 2 demonstrates that differences in the nucleotide sequence in a specific portion determining the ALDH2 activity in the ALDH2 gene-associated site on chromosome 12 can be specifically detected. From the result of Example 2, it is understood that the nucleotide sequence of the target nucleic acid fragment can be detected by the method according to the present invention in which pyrophosphoric acid generated in the progress of the polymerase elongation reaction is detected using a dry analytical element for quantifying pyrophosphoric acid.

### Example 3: Detection of SRY gene-associated site on the short arm of the Y chromosome using a dry analytical element for quantifying inorganic phosphorus

The reflection density (OR_{R}) was measured in the same manner as described above, except that a dry analytical element for quantifying inorganic phosphorus was used which was prepared in the same manner as the preparation of the dry analytical element for quantifying pyrophosphoric acid shown in (1) in Example 1 except that pyrophosphatase was removed from the composition (a) of an aqueous solution for the reagent layer in Table 1, and that 100 µL of reaction solution after PCR was treated with 10 units of pyrophosphatase (pH 7.0, 37°C, 10 minutes). The results were 0.268, 1.268, and 0.273 for the control, sample M, and sample F, respectively.

Example 3 demonstrates that the existence of the target nucleic acid fragment can be specifically detected by the method according to the second embodiment of the present invention in which pyrophosphoric acid generated with the progress in the polymerase elongation reaction is converted into inorganic phosphoric acid with the aid of pyrophosphatase, followed by detection using the dry analytical element for quantifying inorganic phosphorus.

### Example 4: Detection of Pseudomonas aeruginosa in human whole blood using a dry analytical element for quantifying pyrophosphoric acid (experiment employing the examination of Pseudomonas septicemia as a model)

### (1) Preparation of human whole blood containing Pseudomonas aeruginosa

A solution, the concentration of which was varied by diluting the solution with PBS, prepared from the culture solution of *Pseudemonas Syringae*, which was cultured in Luria-Bertani (LB) medium overnight, was added to human whole blood that had been collected in EDTA. Thus, 6-levels of human whole blood containing 0, 5 x 10⁵, 5 x 10⁶, 2.5 x 10⁶, 5 x 10⁷, and 1 x 10⁸ cells per 1 mL, respectively, were prepared. The number of cells is a value estimated using a spectrophotometer.

### (2) Preparation of a dry analytical element for quantifying pyrophosphoric acid

An aqueous solution having the composition (a) shown in Table 4 was coated at the following coverage on a colorless transparent polyethylene terephthalate (PET) smooth film sheet (support) (thickness of 180 µm) having a gelatin undercoating layer. The coating was then dried to provide a reagent layer.

**Table 4**

| Composition (a) of an aqueous solution for the reagent layer | |
|---|---|
| Gelatin | 18.8 g/m² |
| p-Nonylphenoxy polyxydol (glycidol unit: containing 10 units on average) (C₉H₁₉-Ph-O-(CH₂CH(OH)-CH₂-O)₁₀H) | 1.5 g/m² |
| Xanthosine | 1.96 g/m² |
| Peroxidase | 15,000 IU/m² |
| Xanthine oxidase | 13,600 IU/m² |
| Purine nucleoside phosphorylase | 3,400 IU/m² |
| Leuco dye (2-(3,5-dimethoxy-4-hydroxyphenyl)-4-phenethyl-5-(4-dimethylaminophenyl)imidazole | 0.28 g/m² |
| Water (pH was adjusted to 6.8 with a diluted NaOH solution) | 136 g/m² |

On this reagent layer, an aqueous solution for an adhesive layer having the composition (b) shown in Table 5 below was coated at the following coverage. The coating was then dried to provide an adhesive layer. Table 5

| Composition (b) of an aqueous solution for the adhesive layer | |
|---|---|
| Gelatin | 3.1 g/m² |
| p-Nonylphenoxy polyxydol (glycidol unit: containing 10 units on average) (C₉H₁₉-Ph-O-(CH₂CH(OH)-CH₂-O)₁₀H) | 0.25 g/m² |
| Water | 59 g/m² |

Subsequently, water was supplied on the adhesive layer over its whole surface at 30 g/m² to allow the gelatin layer to swell. A broad textile fabric made of genuine polyester was laminated thereon by applying a slight pressure in a substantially even manner to provide a porous developing layer.

An aqueous solution having the composition (c) shown in Table 6 below was then substantially evenly coated on the developing layer at the following coverage. The coating was then dried and cut into a size of 13 mm x 14 mm, and accommodated into a plastic mounting material, thereby preparing a dry analytical element for quantifying pyrophosphoric acid. Table 6

| Composition (c) of an aqueous solution for the developing layer | |
|---|---|
| HEPES | 2.3 g/m² |
| Sucrose | 5.0g/m² |
| Hydroxypropyl methyl cellulose (methoxy group 19 to 24%, hydroxypropoxy group 4 to 12%) | 0.04 g/m² |
| Pyrophosphatase | 14,000 ID/m² |
| Water (pH was adjusted to 7.2 with a diluted NaOH solution) | 98.6 g/m² |

### (3) Relationship between the concentration of pyrophosphoric acid in a sample solution and the coloring of a dry analytical element for quantifying pyrophosphoric acid

Using potassium pyrophosphate (Wako Chemical), the purity of which was confirmed, aqueous solutions of pyrophosphoric acid (0, 0.1, 0.2, 0.5 and 1.0 mM) were prepared, and 20 µl of each of the solutions were spotted onto the dry multi-layered analytical elements prepared in (2) above. The dry analytical elements were incubated for 6 minutes at 37°C, and the optical density of reflection (OD_{R}) was measured at the wavelength of 650 nm from the support side. The results are shown in Table 7. From these results, it is understood that pyrophosphoric acid in a sample solution can be quantitatively measured by using the dry analytical element of the invention. Table 7: Relationship between the concentration of pyrophosphoric acid in a sample solution and the optical density of reflection (OD_{R}) after 5 minutes

| Concentration of pyrophosphoric acid (mM) | Optical density of reflection (OD_{R}) after 5 minutes |
|---|---|
| 0 | 0.36 |
| 0.1 | 0.46 |
| 0.2 | 0.59 |
| 0.5 | 0.80 |
| 1.0 | 1.01 |

### (4) Extraction and purification of nucleic acid from human whole blood

The 6-levels of human whole blood, which were prepared by adding *Pseudomonas aeruginosa* in (1) above, were used as samples. From each sample genomic nucleic acid fragments were extracted and purified using a commercially available kit for extracting and purifying nucleic acids (QIAGEN, QIAamp DNA Blood Mini Kit) and were collected in 1 mL of purified distilled water to prepare a sample solution of nucleic acid containing the target nucleic acid fragment.

### (5) Amplification by PCR

The sample solution of nucleic acid containing the target nucleic acid fragment obtained by extracting and purifying from the 6-levels of human whole blood samples in (3) above was used as it was, and amplification by PCR was carried out under following conditions.

### <Primer>

The following set of primers having a sequence specific to the genomic nucleic acid of *Pseudomonas aeruginosa* (ice nucleation protein (Inak) N-terminus) was used.
Primer (upper): 5'-GCGATGCTGTAATGACTCTCGACAAGC-3'(SEQ ID NO: 5)
Primer (lower): 5'-GGTCTGCAAATTCTGCGGCGTCGTC-3' (SEQ ID NO: 6)

Amplification by PCR was carried out using a reaction solution having the composition below by repeating 30 cycles of denaturing at 94°C for 1 minute, annealing at 55°C for 1 minute, and polymerase elongation reaction at 72°C for 1 minute.

### <Composition of reaction solution>

| | |
|---|---|
| 10× PCR buffer | 5µL |
| 2.5 mM dNTP | 4µL |
| 20 µM primer (upper) | 1µL |
| 20 µM primer (lower) | 1µL |
| Pyrobest | 0.25µL |
| Sample solution of the nucleic acid obtained in (3) | 5µL |
| Purified water | 33.75µL |

### (6) Detection using an analytical element for quantifying pyrophosphoric acid

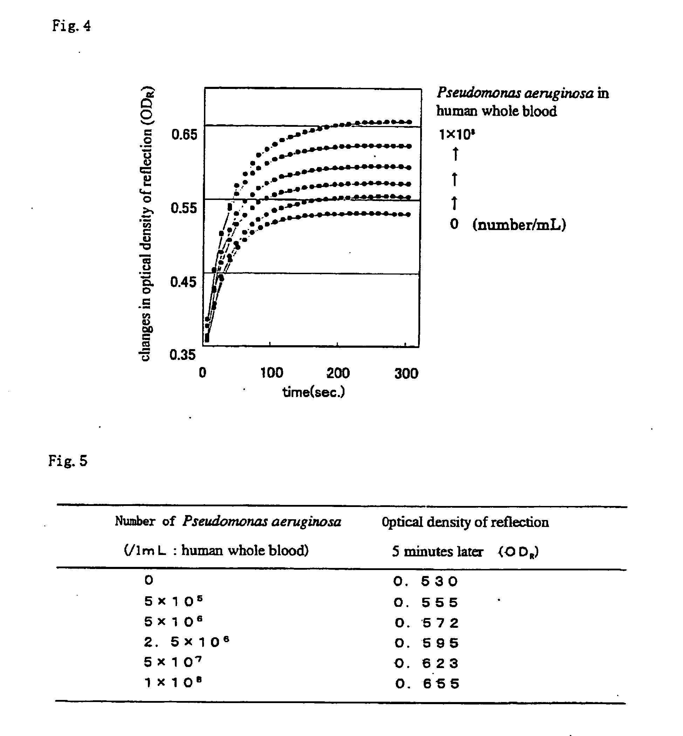

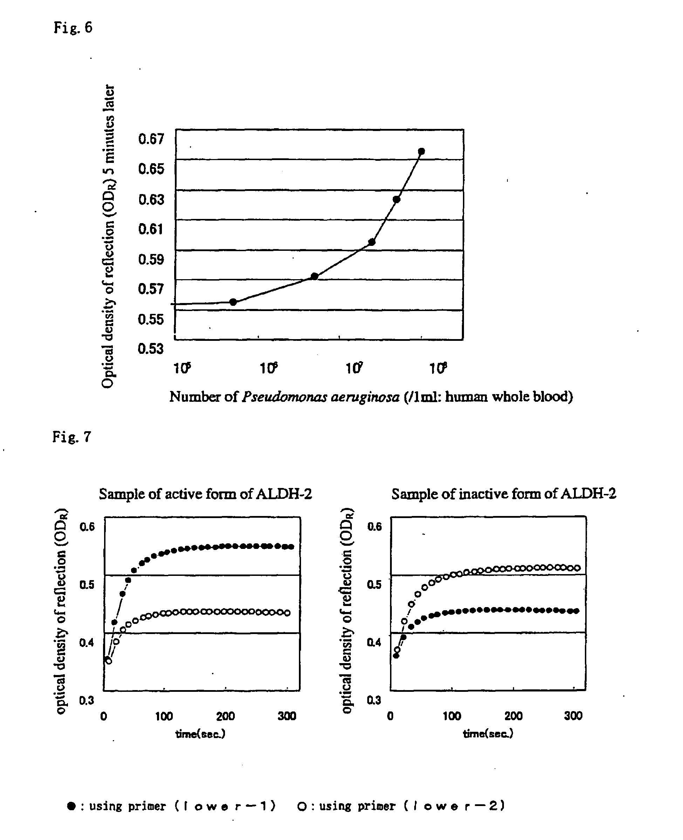

The solutions after being amplified by PCR in (5) above were spotted as they were on the dry analytical element for quantifying pyrophosphoric acid prepared in (2) above in amounts of 20 µL for each solution, and the dry analytical element for quantifying pyrophosphoric acid was incubated at 37°C for 5 minutes. Thereafter, the change with time in the optical density of reflection (OD_{R}) obtained by measuring at a wavelength of 650 nm from the support side is shown in Fig. 4, the optical density of reflection (OD_{R}) is shown in Fig. 5, and the correlation between the number of *Pseudomonas aeruginosa* in human whole blood and the optical density of reflection (OD_{R}) after 5 minutes is shown in Fig. 6.

From the results of Example 4, it is understood that the optical density of reflection (OD_{R}) in accordance with the amount of *Pseudomonas aeruginosa* existing in human whole blood can be obtained by subjecting the sample solution of a nucleic acid containing the target nucleic acid fragment obtained by a conventional method from human whole blood containing *Pseudomonas aeruginosa* to PCR using a set of primers having a sequence specific to the genomic nucleic acid of *Pseudomonas aeruginosa*, using the solution after amplification by PCR as they are, and measuring the generated pyrophosphoric acid as the optical density of reflection (OD_{R}) using the dry analytical element for quantifying pyrophosphoric acid.

### Example 5: Detection of single nucleotide polymorphisms (SNPs) in the aldehyde dehydrogenase gene (ALDH2 gene)-associated site using a dry analytical element for quantifying pyrophosphoric acid (example in which a portion corresponding to single nucleotide polymorphisms is set around the 3' terminus of the primer)

### (1) Preparation of a sample solution of the nucleic acid containing target nucleic acid fragment

From each of the blood specimens collected from each of subjects, who are known to be either in the active form of ALDH2 or in the inactive form of ALDH2 by nucleotide sequencing because of differences in a specific type of nucleotide in the ALDH2 gene-associated site, genomic nucleic acid fragments were extracted and purified using a commercially available kit for extracting and purifying nucleic acids (QIAGEN, QIAamp DNA Blood Mini Kit). The genomic nucleic acid fragments were then collected into 1 mL of purified distilled water, thereby preparing sample solutions of the nucleic acid containing the target nucleic acid fragment.

### (2) Preparation of a dry analytical element for quantifying pyrophosphoric acid

A dry analytical element for quantifying pyrophosphoric acid was prepared by the method described in Example 4.

### (3) Amplification by PCR

Sample solutions of the nucleic acid containing the target nucleic acid fragments obtained in (1) above by extracting and purifying human whole blood samples either in the active form of ALDH2 or in the inactive form of ALDH2 were used as they were, and the amplification by PCR was carried out under the following conditions.

### <Primer>

A set of a primer (upper) common in the ALDH2 gene-associated site on chromosome 12, and two primers, i.e., a primer (lower-1) and a primer (lower-2) corresponding to the active form and the inactive form of ALDH2, respectively, in which a portion corresponding to single nucleotide polymorphisms determining the ALDH2 activity is set around the 3'-terminus (underlined portion of the primer nucleotide sequence described in lower-1 and lower-2), were used.
Primer (upper): 5'-AACGAAGCCCAGCAAATGA-3' (SEQ ID NO: 7)
Primer (lower-1): 5'-GGGCTGCAGGCATACACAGA-3' (SEQ ID NO: 8)
Or,
Primer (upper): 5'-AACGAAGCCCAGCAAATGA-3' (SEQ ID NO: 9)
Primer (lower-2): 5'-GGGCTGCAGGCATACACAAA-3' (SEQ NO: 10)

Amplification by PCR was carried out using a reaction solution having the composition below by repeating 35 cycles of denaturing at 94°C for 20 seconds, annealing at 60°C for 30 seconds, and polymerase elongation reaction at 72°C for 1 minute and 30 seconds.

### <Composition of reaction solution>

| | |
|---|---|
| 10× PCR buffer | 5 µL |
| 2.5 mM dNTP | 5µL |
| 5 µM primer (upper) | 2µL |
| 5 µM primer (lower-1 or lower-2) | 2µL |
| Taq | 0.5 µL |
| Sample solution of the nucleic acid fragment obtained in (1) | 0.5µL |
| Purified water | 35µL |

### (4) Detection using an analytical element for quantifying pyrophosphoric acid

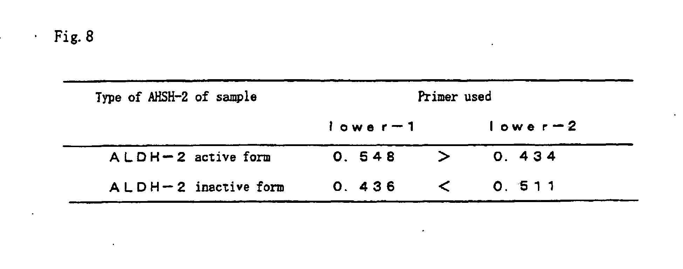

The solutions after amplification by PCR in (3) above were spotted as they were on the dry analytical element for quantifying pyrophosphoric acid prepared in (2) above in amounts of 20 µL for each solution, and the dry analytical element for quantifying pyrophosphoric acid was incubated at 37°C for 5 minutes. Thereafter, the change with time in the optical density of reflection (OD_{R}) obtained by measuring at a wavelength of 650 nm from the support side is shown in Fig. 7, and the optical density of reflection (OD_{R}) after 5 minutes is shown in Fig. 8.

From the results of Example 5, it is understood that the active form of ALDH2 of the sample, i.e., single nucleotide polymorphisms (SNPs) of an aldehyde dehydrogenase gene (ALDH2 gene)-associated site can be detected by performing PCR using a primer set comprising a common primer and either one of two types of primers corresponding to the active form or the inactive form of ALDH2, respectively. In those two primers, there is a portion corresponding to the single nucleotide polymorphisms determining the ALDH2 activity around the 3'-terminus. The solution after amplifying by PCR is used as it is. The amount of the pyrophosphoric acid generated is measured as the increase/decrease in the optical density of reflection (OD_{R}) using the dry analytical element for quantifying pyrophosphoric acid. The correlation between the size of the optical density of reflection (OD_{R}) and two types of primers corresponding to the active form and the inactive form of ALDH2 used is determined.

### Industrial Applicability

The present invention provides a simple and swift method and a kit for analyzing a target nucleic acid fragment having a specific nucleotide sequence, which is effective in, for example, the clinical examination of infectious diseases caused by viruses, bacteria etc., and the examination of genetic diseases resulting from genetic features of individuals. Further, by using a dry analytical element for quantifying pyrophosphoric acid which comprises the reagents for quantification of pyrophosphoric acid according to the present invention, pyrophosphoric acid can be conveniently and quickly quantified in a simple device for colorimetry.

## Claims

1. A dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus, which comprises a reagent layer containing xanthosine or inosine, pyrophosphatase, purine nucleoside phosphorylase, xanthine oxidase, peroxidase, and a color developer.

2. A method for analyzing a target nucleic acid fragment which comprises steps of reacting a target nucleic acid fragment, at least a part of its nucleotide sequence being known, at least one primer complementary with a part of the target nucleic acid fragment, at least one deoxynucleoside triphosphate (dNTP) and at least one polymerase, and detecting occurrence of the progress on polymerase elongation reaction using the target nucleic acid fragment as template and starting from the 3' terminus of the primer, wherein whether or not polymerase elongation reaction is proceeded is detected by detecting pyrophosphoric acid generated upon said polymerase elongation reaction, wherein detection of pyrophosphoric acid is carried out using colorimetry and a dry analytical element according to claim 1.

3. A method for analyzing a target nucleic acid fragment according to claim 2, wherein the dry analytical element is a dry analytical element for quantifying inorganic phosphorus which comprises a reagent layer containing xanthosine or inosine, purine nucleoside phosphorylase, xanthine oxidase, peroxidase, and a color developer, after pyrophosphoric acid is enzymatically converted into inorganic phosphoric acid.

4. A method for analyzing a target nucleic acid fragment according to claim 3, wherein the polymerase is selected from the group consisting of DNA polymerase I, Klenow fragment of DNA polymerase I, Bst DNA polymerase, and reverse transcriptase.

5. A method for analyzing a target nucleic acid fragment according to claim 2, wherein the polymerase is selected from the group consisting of DNA polymerase I, Klenow fragment of DNA polymerase I, Bst DNA polymerase, and reverse transcriptase.

6. A method for analyzing a target nucleic acid fragment according to claim 2, wherein an enzyme for converting pyrophosphoric acid into inorganic phosphoric acid is pyrophosphatase.

7. A method for analyzing a target nucleic acid fragment according to claim 2, wherein the analysis of a target nucleic acid is a detection of the presence or abundance of the target nucleic acid fragment, or a detection of the nucleotide sequence of the target nucleic acid fragment.

8. A method for analyzing a target nucleic acid fragment according to claim 7, wherein the detection of the nucleotide sequence of the target nucleic acid fragment is a detection of mutation or polymorphisms of the target nucleic acid fragment.

9. A kit which comprises at least one primer complementary with a part of the target nucleic acid fragment to be analyzed, at least one deoxynucleoside triphosphate, at least one polymerase, and a dry analytical element according to claim 1 for quantifying pyrophosphoric acid.

10. A kit according to claim 9 further comprising pyrophoshatase .

## Patentansprüche

1. Trockenes analytisches Element zum Quantifizieren von Pyrophosphorsäure, welches ein Reagens zum Umwandeln von Pyrophosphorsäure in anorganischen Phosphor und eine Reagensschicht umfasst, die eine Reagensgruppe zum Durchführen einer Farbreaktion in Abhängigkeit von der Menge an anorganischem Phosphor enthält, umfassend eine Reagensschicht, die Xanthosin oder Inosin, Pyrophosphatase, Purinnucleosidphosphatase, Xanthinoxidase, Peroxidase und ein Farbentwicklungsmittel enthält.

2. Verfahren zum Analysieren eines Zielnucleinsäurefragmentes, welches die Stufen des Umsetzens eines Zielnucleinsäurefragmentes, wobei mindestens ein Teil seiner Nucleotidsequenz bekannt ist, mindestens eines Primers, der zu einem Teil des Zielnucleinsäurefragmentes komplementär ist, mindestens eines Desoxynucleosidtriphosphats (dNTP) und mindestens einer Polymerase, und des Detektierens des Auftretens des Fortschreitens bei der Polymeraseverlängerungsreaktion unter Verwendung des Zielnucleinsäurefragmentes als Matrize und ausgehend vom 3'-Terminus des Primers, wobei, ob die Polymeraseverlängerungsreaktion fortschreitet oder nicht, durch Detektieren von Pyrophosphorsäure, die bei der Polymeraseverlängerungsreaktion erzeugt wird, detektiert wird, wobei die Detektion von Pyrophosphorsäure unter Verwendung von Kolorimetrie und eines trockenen analytischen Elements gemäß Anspruch 1 durchgeführt wird, umfasst.

3. Verfahren zum Analysieren eines Zielnucleinsäurefragmentes gemäß Anspruch 2, wobei das trockene analytische Element ein trockenes analytisches Element zum Quantifizieren von anorganischem Phosphor ist, welches eine Reagensschicht umfasst, enthaltend Xanthosin oder Inosin, Purinnucleosidphosphorylase, Xanthinoxidase, Peroxidase und ein Farbentwicklungsmittel, nachdem Pyrophosphorsäure enzymatisch in anorganische Phosphorsäure umgewandelt wurde.

4. Verfahren zum Analysieren eines Zielnucleinsäurefragmentes gemäß Anspruch 3, wobei die Polymerase ausgewählt ist aus der Gruppe, bestehend aus DNA-Polymerase I, Klenow-Fragment der DNA-Polymerase I, Bst-DNA-Polymerase und reverser Transkriptase.

5. Verfahren zum Analysieren eines Zielnucleinsäurefragmentes gemäß Anspruch 2, wobei die Polymerase ausgewählt ist aus der Gruppe, bestehend aus DNA-Polymerase I, Klenow-Fragment der DNA-Polymerase I, Bst-DNA-Polymerase und reverser Transkriptase.

6. Verfahren zum Analysieren eines Zielnucleinsäurefragmentes gemäß Anspruch 2, wobei ein Enzym zum Umwandeln von Pyrophosphorsäure in anorganische Phosphorsäure Pyrophosphatase ist.

7. Verfahren zum Analysieren eines Zielnucleinsäurefragmentes gemäß Anspruch 2, wobei die Analyse einer Zielnucleinsäure eine Detektion des Vorhandenseins oder der Menge des Zielnucleinsäurefragmentes oder eine Detektion der Nucleotidsequenz des Zielnucleinsäurefragmentes ist.

8. Verfahren zum Analysieren eines Zielnucleinsäurefragmentes gemäß Anspruch 7, wobei die Detektion der Nucleotidsequenz des Zielnucleinsäurefragmentes eine Detektion von Mutation oder Polymorphismen des Zielnucleinsäurefragmentes ist.

9. Kit, der wenigstens einen Primer, der zu einem Teil des Zielnucleinsäurefragmentes, das analysiert werden soll, komplementär ist, mindestens ein Desoxynucleosidtriphosphat, mindestens eine Polymerase und ein trockenes analytisches Element gemäß Anspruch 1 zum Quantifizieren von Pyrophosphorsäure umfasst.

10. Kit gemäß Anspruch 9, weiterhin umfassend Pyrophosphatase.

## Revendications

1. Un élément analytique sec pour la quantification d'acide pyrophosphorique qui comprend un réactif pour transformer l'acide pyrophosphorique en phosphore inorganique et une couche de réactif contenant un groupe de réactif pour effectuer une réaction de couleur dépendant de la quantité de phosphore inorganique qui comprend une couche de réactif contenant de la xanthosine ou de l'inosine, de la pyrophosphatase, de la purine nucléoside phosphorylase, de la xanthine oxydase, de la peroxydase et d'un révélateur de couleur.

2. Un procédé d'analyse d'un fragment d'acide nucléique cible comprenant les étapes consistant à faire réagir un fragment d'acide nucléique cible, au moins une partie de sa séquence de nucléotide étant connue, au moins une amorce complémentaire d'une partie du fragment d'acide nucléique cible, au moins un désoxynucléoside triphosphate (dNTP) et au moins une polymérase, et à détecter l'occurrence du progrès de la réaction d'extension par la polymérase en utilisant le fragment d'acide nucléique cible en tant que matrice et en commençant à partir de l'extrémité 3' de l'amorce, dans lequel on détecte si la réaction d'extension par la polymérase est procédée ou non en détectant l'acide pyrophosphorique généré par la réaction d'extension par la polymérase, dans lequel la détection de l'acide pyrophosphorique est effectuée en utilisant la colorimétrie et un élément analytique sec selon la revendication 1.

3. Le procédé d'analyse d'un fragment d'acide nucléique cible selon la revendication 2, dans lequel l'élément analytique sec est un élément analytique sec pour la quantification de phosphore inorganique, comprenant une couche de réactif contenant de la xanthosine ou de l'inosine, de la purine nucléoside phosphorylase, de la xanthine oxydase, de la peroxydase et un révélateur de couleur, après la transformation enzymatique de l'acide pyrophosphorique en acide phosphorique inorganique.

4. Le procédé d'analyse d'un fragment d'acide nucléique cible selon la revendication 3, dans lequel la polymérase est choisie parmi le groupe consistant en ADN polymérase I, fragment de Klenow de l'ADN polymérase I, Bst ADN polymérase et transcriptase inverse.

5. Le procédé d'analyse d'un fragment d'acide nucléique cible selon la revendication 2, dans lequel la polymérase est choisie parmi le groupe consistant en ADN polymérase I, fragment de Klenow de l'ADN polymérase I, Bst ADN polymérase et transcriptase inverse.

6. Le procédé d'analyse d'un fragment d'acide nucléique cible selon la revendication 2, dans lequel une enzyme pour transformer l'acide pyrophosphorique en acide phosphorique inorganique est la pyrophosphatase.

7. Le procédé d'analyse d'un fragment d'acide nucléique cible selon la revendication 2, dans lequel l'analyse d'un nucléique acide cible est la détection de la présence ou de l'abondance du fragment d'acide nucléique cible ou la détection de la séquence de nucléotide du fragment d'acide nucléique cible.

8. Le procédé d'analyse d'un fragment d'acide nucléique cible selon la revendication 7, dans lequel la détection de la séquence de nucléotide du fragment d'acide nucléique cible est la détection d'une mutation ou des polymorphismes du fragment d'acide nucléique cible.

9. Une trousse comprenant au moins une amorce complémentaire d'une partie du fragment d'acide nucléique cible à analyser, au moins un désoxynucléoside triphosphate, au moins une polymérase et un élément analytique sec selon la revendication 1 pour la quantification d'acide pyrophosphorique.

10. La trousse selon la revendication 9, comprenant en outre de la pyrophosphatase.
